(19) Europäisches Patentamt
European Patent Office
Office européen des brevets

(11) Numéro de publication : **0 433 132 B1**

# (12) FASCICULE DE BREVET EUROPEEN

(45) Date de publication du fascicule du brevet : **05.01.94 Bulletin 94/01**

(51) Int. Cl.$^5$ : **A61K 7/00,** A61K 9/127, A61K 7/06, A61K 7/48

(21) Numéro de dépôt : **90403450.1**

(22) Date de dépôt : **05.12.90**

## (54) Composition cosmétique pour application topique contenant des huiles essentielles.

(30) Priorité : **13.12.89 FR 8916479**

(43) Date de publication de la demande : **19.06.91 Bulletin 91/25**

(45) Mention de la délivrance du brevet : **05.01.94 Bulletin 94/01**

(84) Etats contractants désignés : **AT BE CH DE DK ES GB GR IT LI NL SE**

(56) Documents cités :
**EP-A- 0 347 306**
**FR-A- 2 485 921**
**FR-A- 2 490 504**

(73) Titulaire : **L'OREAL**
**14, Rue Royale**
**F-75008 Paris (FR)**

(72) Inventeur : **Richoux, Isabelle**
**19, rue Boursault**
**F-75017 Paris (FR)**

(74) Mandataire : **Peuscet, Jacques et al**
**Cabinet Peuscet 68, rue d'Hauteville**
**F-75010 Paris (FR)**

EP 0 433 132 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des compositions cosmétiques pour application topique contenant des huiles essentielles ayant une action spécifique en plus de l'activité parfumante éventuelle.

Les huiles essentielles naturelles sont des produits obtenus à partir de matières premières d'origine végétale (feuilles, tiges, fleurs, rameaux de plantes et/ou plantes entières), soit par entraînement à la vapeur d'eau, sèche ou humide, soit par des procédés mécaniques, soit par distillation à sec, soit par extraction au moyen de solvants volatils (voir la norme NF-T-75006). Les huiles essentielles se distinguent des huiles végétales par le fait qu'elles ne peuvent pas être décomposées par saponification en glycérol et savon d'acide gras. Ces huiles essentielles peuvent être également préparées par synthèse.

Les huiles essentielles sont souvent utilisées pour leur odeur, mais également pour leurs nombreuses autres activités pharmaceutiques et/ou cosmétiques, qui sont connues de très longue date (voir notamment Aromathérapie par J. Valuet, Edition Maloine, 1984).

Les huiles essentielles étaient, jusqu'à présent, généralement utilisées sous forme de solution hydroalcoolique. Or, il est bien connu que l'éthanol est irritant pour la peau et que l'application répétée de solution hydroalcoolique d'huile essentielle peut provoquer un dessèchement de la peau ou des rougeurs.

Il est, par ailleurs, connu que certains lipides amphiphiles, ioniques ou non-ioniques, sont susceptibles de former en présence d'une phase aqueuse une phase lamellaire puis, par agitation, des vésicules en dispersion dans ladite phase aqueuse. Ces vésicules sont formées d'un ou, le plus souvent, de plusieurs feuillet(s) concentrique(s) (couches bimoléculaires ou multimoléculaires) de lipides encapsulant une phase aqueuse. De telles dispersions de vésicules ainsi que certains de leurs modes de préparation sont, par exemple, décrits dans les brevets FR-A-2 315 991 et FR-A-2 543 018. Il est également connu, par les brevets français 2 485 921 et 2 490 504, que les vésicules de lipides amphiphiles stabilisent en phase aqueuse des dispersions de liquides non-miscibles à l'eau, sans qu'il soit nécessaire d'ajouter un agent émulsifiant.

La présente invention est l'application de cette propriété des vésicules de lipides amphiphiles pour la préparation de compositions cosmétiques pour application topique contenant des huiles essentielles ayant une activité antibactérienne, antifongique ou insectifuge en plus de l'activité parfumante éventuelle.

La présente invention a pour objet une composition cosmétique pour application topique contenant des huiles essentielles ayant une activité autre que parfumante, caractérisée par le fait qu'elle contient, dans une phase aqueuse continue de dispersion :

a) des vésicules préparées à partir d'une phase lipidique comprenant au moins un lipide amphiphile, ionique ou non-ionique, éventuellement associé à au moins un additif stabilisant, et

b) au moins une huile essentielle, naturelle ou synthétique, sous forme de gouttelettes dispersées dans la phase aqueuse de dispersion, ladite (ou lesdites) huile(s) essentielle(s) étant présente(s) à raison de 0,1 à 16 % en poids par rapport au poids total de la composition et le rapport pondéral de la phase lipidique à l'(ou aux) huile(s) essentielle(s) étant supérieur à 0,2 et inférieur ou égal à 4.

Selon l'invention, d'une part, les vésicules ont, avantageusement, une dimension moyenne comprise entre 10 et 1000 nm et, d'autre part, les gouttelettes d'huile(s) essentielle(s) ont une dimension moyenne comprise entre 100 et 10000 nm.

Selon la présente invention, on a trouvé que l'activité autre que parfumante des huiles essentielles était renforcée par la présence des vésicules et qu'elle était plus durable dans le temps qu'en l'absence de vésicules. De plus, on a constaté que, lorsque le rapport pondéral (phase lipidique/huile(s) essentielle(s)) est inférieur à 0,2, dans le cas de certaines applications, par exemple comme déodorant, la composition est irritante. On a également constaté que, lorsque ce rapport est supérieur à 4, la composition devient collante et, par conséquent, non cosmétique.

Les compositions selon l'invention ont également l'avantage d'être non grasses et d'avoir une action adoucissante sur la peau.

Selon un premier mode de réalisation de l'invention, l'huile essentielle contenue dans la composition est choisie parmi celles ayant une activité antibactérienne et/ou antifongique. Les compositions selon l'invention, contenant des huiles essentielles antibactériennes et/ou antifongiques, peuvent être utilisées largement en dermatologie. De plus, les compositions contenant des huiles essentielles ayant une activité antibactérienne vis-à-vis des bactéries Gram-positif sont particulièrement avantageuses, car elles peuvent être utilisées comme déodorant ; il est connu, en effet, que les bactéries Gram-positif sont principalement responsables de la mauvaise odeur de la transpiration (voir, à ce sujet, Najir Shehadeh et autres, J. of Invest. Derm., 1963, Vol. 41, page 3 et J.J. Leyden et autres, J. of Invest. Derm., 1981, Vol. 77, pages 413 - 417).

L'huile essentielle ayant une activité antibactérienne et/ou antifongique est choisie, de préférence, dans le groupe formé par les huiles de litsea cubeba, de sarriette, de vétivers (Bourbon, Java, Palmarosa et Haïti), de santal, de melaleuca, de camomille, de bois de rose du Brésil, de cannelle (Ceylan et Chine), de zeste de

citron, de géranium (Bourbon et Palmarosa), de lavandin, de menthe poivrée, de thym, de patchouli, de linalol et de coriandre. Parmi ces huiles essentielles, on préfère la sarriette, le vétiver, le litsea cubeba, le melaleuca, notamment le melaleuca alternifolia, et particulièrement la sarriette et le litsea cubeba. Les huiles essentielles telles que le bois de rose du Brésil, la cannelle de Ceylan et de Chine, le zeste de citron, le lavandin, la menthe poivrée, le thym et le linalol, ont une activité antibactérienne sur les bactéries Gram-positif ; le patchouli a une activité antifongique ; les huiles de sarriette, de litsea cubeba, de vétivers ou de santal ont à la fois une activité antibactérienne et une activité antifongique.

L'huile essentielle à activité antifongique et/ou antibactérienne est, de préférence, présente dans la composition à raison de 0,2 à 10 % en poids par rapport au poids total de la composition.

Selon un second mode de réalisation de l'invention, l'huile essentielle contenue dans la composition est choisie parmi celles ayant une activité insectifuge, par exemple anti-moustique. L'huile essentielle est, de préférence, choisie parmi les huiles de citronnelle, de géranium, de lavande, de lavandin, de pins, d'eucalyptus et de laurier noble.

Dans les compositions insectifuges selon l'invention, les huiles essentielles sont, de préférence, présentes à raison de 1 à 12 % en poids par rapport au poids total de la composition.

Les lipides amphiphiles formant les vésicules sont des lipides ioniques ou non-ioniques ; bien entendu, on peut aussi utiliser un melange de lipide(s) ionique(s) et non-ionique(s). Parmi les lipides ioniques utilisables, on peut citer les phospholipides naturels, tels que les lécithines d'oeuf et de soja et la sphingomyéline, et les phospholipides de synthèse, tels que le dipalmitoylphosphatidylcholine ou la lécithine hydrogénée.

Le lipide amphiphile présent dans la phase lipidique formant des vésicules est, de préférence, choisi parmi les lipides amphiphiles non-ioniques. Il est plus particulièrement choisi parmi les dérivés de polyglycérol, linéaires ou ramifiés, de formule (I) :

$$RO-\left[C_3H_5(OH)O\right]_{\bar{n}}-H \qquad (I)$$

formule dans laquelle :

- $C_3H_5(OH)O$- représente les structures suivantes prises en mélange ou séparément :

$-CH_2-CHOH-CH_2O-$

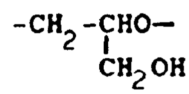

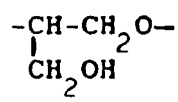

- $\bar{n}$ est une valeur statistique moyenne comprise entre 2 et 6 et
- R est :

  a) soit une chaîne aliphatique $R_1$ ou un reste $R_2CO$, $R_1$ étant un radical aliphatique, linéaire ou ramifié, en $C_{12}$ - $C_{18}$ et $R_2$ un radical aliphatique, linéaire ou ramifié, en $C_{11}$ - $C_{17}$ ;

  b) soit

$$R_3\left[O-C_2H_3(R_4)\right]-$$

où $-O-C_2H_3(R_4)-$ représente les structures suivantes prises en mélange ou séparément :

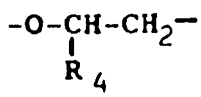

et

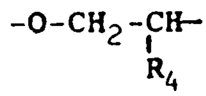

$R_3$ étant un radical $R_1$ ou $R_2CO$,
$R_4$ étant un radical $R_1$ , et
$R_1$ et $R_2$ ayant les significations données ci-dessus.

L'additif stabilisant associé à la phase lipidique est destiné, de façon connue, à modifier la perméabilité et/ou la charge superficielle des vésicules. Il est choisi, de préférence, dans le groupe formé par les stérols et les stabilisants anioniques. Le stérol est plus particulièrement le cholestérol. Le stabilisant anionique est plus particulièrement choisi dans le groupe formé par les esters phosphoriques d'alcools gras en $C_{12}$-$C_{22}$ et les sels mono- et disodiques d'acyl-glutamates, le radical acyle étant en $C_{14}$-$C_{22}$. On peut citer, par exemple, le sel monosodique du stéaroyl-glutamate. De façon connue, on peut utiliser, à la fois, un stérol et un stabilisant anionique.

La phase lipidique représente avantageusement de 0,5 à 16 % en poids par rapport au poids total de la composition, et, de préférence, de 2 à 10 % en poids.

Le(s) lipide(s) amphiphile(s) ionique(s) ou non-ionique(s) de la phase lipidique représente(nt), de préférence, de 0,2 à 16 % en poids du poids total de la composition et, mieux encore, de 1 à 10 %.

L'additif stabilisant est, de préférence, présent dans la phase lipidique à raison de moins de 10 % en poids par rapport au poids total de la composition.

Des additifs cosmétiques sont éventuellement présents dans la phase aqueuse de dispersion ; ils peuvent être pris dans le groupe formé par les conservateurs, les parfums, les agents acidifiants, les agents alcalinisants, les stabilisants, les colorants et les agents épaississants. L'agent épaississant peut, notamment, être choisi dans le groupe formé par les dérivés cellulosiques, la gomme de xanthane et les acides polyacryliques réticulés, en particulier ceux vendus sous la dénomination commerciale "CARBOPOL"®, par exemple "CARBOPOL 934"® et "CARBOPOL 940"®, par la société "GOODRICH".

On peut ajouter également à la composition cosmétique un antibactérien et/ou un antifongique autre qu'une huile essentielle ou un insectifuge autre qu'une huile essentielle ; on peut, par exemple, pour les compositions insectifuges, introduire, dans la phase aqueuse de dispersion, un antimoustique de synthèse tel que le N,N-diéthyl-méthyl-3-benzamide.

Les compositions cosmétiques selon l'invention peuvent se présenter sous la forme de liquides plus ou moins épaissis, de gels, de crèmes, de sticks ou d'aérosols.

Les exemples donnés ci-dessous, à titre illustratif, permettront de mieux comprendre l'invention.

<u>EXEMPLE 1</u> : <u>COMPOSITION INSECTIFUGE</u>

Dans une première étape, on fond, en agitant doucement à une température de 80 - 90°C, un mélange de 3,8 g de lipide non-ionique de formule (II) :

$$C_{16}H_{33}-O-\left[C_3H_5(OH)-O\right]_{\bar{n}}-H \qquad (II)$$

formule dans laquelle :

. $-C_3H_5(OH)O-$ représente un mélange des structures :

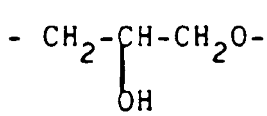 , ; 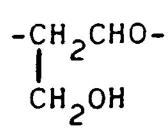

et

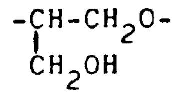

. $\bar{n}$ est une valeur statistique moyenne égale à 3, avec 3,8 g de cholestérol et 0,4 g de dicétylphosphate.

On introduit dans le mélange fondu 16 g d'eau portée à 90°C contenant un conservateur et l'on mélange pendant environ 6 à 8 minutes au moyen d'un agitateur "ULTRA TURRAX"®. A la phase ainsi obtenue, on ajoute 24 g d'eau à température ambiante, puis on homogénéise le mélange à l'agitateur "ULTRA TURRAX"® pendant encore environ 6 à 8 minutes avant de le laisser revenir à la température ambiante. On obtient ainsi une dispersion de vésicules dans une phase aqueuse continue.

Dans une seconde étape, on additionne goutte à goutte à la phase aqueuse 10 g d'huile essentielle de géranium en agitant à l'agitateur "ULTRA TURRAX"® pendant 5 minutes. Puis on ajoute un gel aqueux bien homogène constitué de 0,3 g d'agent epaississant vendu sous la dénomination commerciale "CARBOPOL 934"® par la société "GOODRICH", dissous dans 6 g d'eau renfermant des conservateurs et ajusté à pH 6,5 avec de l'amino-2 méthyl-2 propanol-1 et on complète à 100 g avec de l'eau.

On obtient une composition sous forme d'une crème stable. Cette crème répartie sur la peau a un effet antimoustique de grande intensité.

EXEMPLE 2 : COMPOSITION INSECTIFUGE

On prépare une composition comme dans l'exemple 1, en remplaçant le dicétylphosphate par le sel monosodique du glutamate de formule :

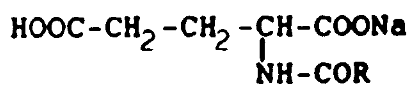

dans laquelle R est un mélange de radicaux alkényle et/ou alkyle en $C_{13}$ - $C_{21}$ dérivé des acides gras du suif, vendu sous la dénomination commerciale "ACYLGLUTAMATE HS 11"® par la société "AJINOMOTO".

On obtient une crème ayant les mêmes propriétés que celle de l'exemple 1.

EXEMPLE 3 : COMPOSITION INSECTIFUGE

Dans une première étape, on fond, en agitant doucement à une température de 80 - 90°C, un mélange de 1,8 g de lipide non-ionique de formule :

$$C_{16}H_{33}-[OCH_2-CHOH-CH_2]_{\bar{n}}-OH$$

où $\bar{n}$ est une valeur statistique moyenne égale à 3, avec 1,8 g de cholestérol et 0,2 g de dicétylphosphate.

On introduit dans le mélange fondu 7,6 g d'eau portée à 90°C contenant un conservateur et l'on mélange pendant environ 6 à 8 minutes au moyen d'un agitateur "ULTRA TURRAX"®. A la phase ainsi obtenue, on ajoute 11,4 g d'eau à température ambiante, puis on homogénéise le mélange à l'agitateur "ULTRA TURRAX"® pendant encore environ 6 à 8 minutes avant de laisser revenir le mélange à la température ambiante. On obtient une dispersion de vésicules dans une phase aqueuse.

Dans une seconde étape, on additionne, à la phase aqueuse, goutte à goutte, 4 g d'huile essentielle de citronnelle en agitant à l'agitateur "ULTRA TURRAX"® pendant 5 minutes. Puis on ajoute un gel aqueux bien homogène constitué de 0,15 g de "CARBOPOL 934"® vendu par la société "GOODRICH", dissous dans 6 g d'eau renfermant des conservateurs et ajusté à pH 6,5 avec de l'amino-2 méthyl-2 propanol-1 et on complète à 100 g avec de l'eau.

On obtient une crème ayant les memes propriétés que celle de l'exemple 1.

EXEMPLE 4 : COMPOSITION INSECTIFUGE

On prépare une composition comme dans l'exemple 3, sauf que le lipide non-ionique utilisé a pour formule :

$$C_{15}H_{31}-CO-\left[OCH_2-CHOH-CH_2\right]_2-OH$$

On obtient une crème ayant les mêmes propriétés que celle de l'exemple 1.

EXEMPLE 5 : COMPOSITION INSECTIFUGE

Dans une première étape, on fond, en agitant doucement à une température de 80 - 90°C, un mélange de 6 g de lipide non-ionique de formule :

$$C_{12}H_{25}-\left[OC_2H_3(R_4)\right]-O-\left[C_3H_5-(OH)-O\right]_{\bar{n}}-H$$

formule dans laquelle :

- $C_3H_5(OH)O$- est constitué par un mélange des structures :

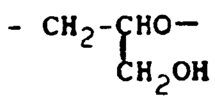

et

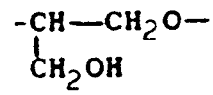

- O-$C_2H_3(R_4)$- est constitué par un mélange des structures :

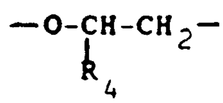

et

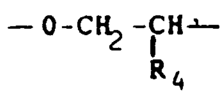

- $\bar{n} = 6$
- $R_4$ est un mélange de radicaux $C_{14}H_{29}$ et $C_{16}H_{33}$, avec 1,6 g de cholestérol.

On introduit dans le mélange fondu 15,2 g d'eau portée à 90°C contenant un conservateur et l'on mélange pendant environ 6 à 8 minutes au moyen d'un agitateur "ULTRA TURRAX"®. A la phase ainsi obtenue, on ajoute 22,8 g d'eau à température ambiante, puis on homogénéise le mélange à l'agitateur "ULTRA TURRAX"® pendant encore environ 6 à 8 minutes avant de laisser revenir le mélange à la température ambiante. On obtient une dispersion de vésicules dans une phase aqueuse.

Dans une seconde étape, on additionne la phase aqueuse, goutte à goutte, 10 g d'huile essentielle de géranium en agitant à l'agitateur "ULTRA TURRAX"® pendant 5 minutes. Puis on ajoute un gel aqueux bien homogène constitué de 0,3 g de "CARBOPOL 934"® vendu par la société "GOODRICH", dissous dans 6 g d'eau renfermant des conservateurs et ajusté à pH 6,5 avec de l'amino-2 méthyl-2 propanol-l et on complète à 100 g avec de l'eau.

On obtient une crème ayant les mêmes propriétés que celle de l'exemple 1.

EXEMPLE 6 : COMPOSITION INSECTIFUGE

On prépare une composition comme dans l'exemple 5 en remplaçant l'huile de géranium par de l'huile de citronnelle.

On obtient une crème ayant les mêmes propriétés que celle de l'exemple 1.

EXEMPLE 7 : COMPOSITION DEODORANTE

Dans une première étape, on fond, en agitant doucement à une température de 80 - 90°C, un mélange de 1,9 g de lipide non-ionique de formule :

$$C_{16}H_{33}-[OC_3H_5(OH)]_{\bar{n}}-OH$$

où $-OC_3H_5(OH)-$ est constitué par un mélange des radicaux

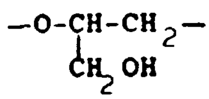

et

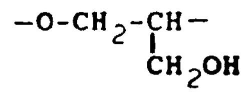

où $\bar{n}$ est une valeur statistique moyenne égale à 3, avec 1,9 g de cholestérol et 0,2 g de dicétylphosphate.

On introduit dans le mélange fondu 8 g d'eau portée à 90°C contenant un conservateur et l'on mélange pendant environ 6 à 8 minutes au moyen d'un agitateur "ULTRA TURRAX"®. A la phase ainsi obtenue, on ajoute 12 g d'eau à température ambiante, puis on homogénéise le mélange à l'agitateur "ULTRA TURRAX"® pendant encore environ 6 à 8 minutes avant de laisser revenir le mélange à la température ambiante. On obtient des vésicules de lipides dispersés dans une phase aqueuse.

Dans une seconde étape, on additionne, goutte à goutte 2 g d'huile essentielle de litsea cubeba en agitant à l'agitateur "ULTRA TURRAX"® pendant 5 minutes. Puis on ajoute un gel aqueux bien homogène constitué de 0,15 g de "CARBOPOL 940"® vendu par la société "GOODRICH", dissous dans 3 g d'eau renfermant des conservateurs et ajusté à pH 6 avec de la triéthanolamine ; on complète à 100 g avec de l'eau.

On obtient une composition sous forme de crème ; lorsque l'on applique cette crème sur la peau, on constate un effet antibactérien et antifongique de grande intensité.

EXEMPLE 8 : COMPOSITION DEODORANTE

On prépare une composition comme dans l'exemple 1, en remplaçant l'huile de litsea cubeba par de l'huile de sarriette. La crème obtenue a les mêmes propriétés que celle de l'exemple 7.

EXEMPLE 9 : COMPOSITION DEODORANTE

On prépare une composition comme dans l'exemple 1, en remplaçant l'huile de litsea cubeba par un mélange sarriette/litsea cubeba (1,5 g/0,5 g).

La crème obtenue a les mêmes propriétés que celle de l'exemple 7.

EXEMPLE 10 : COMPOSITION DEODORANTE

On prépare une composition comme dans l'exemple 7, en remplaçant le dicétylphosphate par du sel monosodique du glutamate de formule :

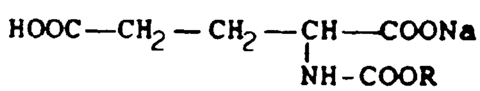

formule dans laquelle R est un mélange de radicaux alkényle et/ou alkyle en $C_{13}$- $C_{21}$ dérivé des acides gras du suif, vendu la dénomination commerciale "ACYLGLUTAMATE HS 11"® par la société "AJINOMOTO" et en remplaçant l'huile essentielle de litsea cubeba par un mélange sarriette/vétiver Bourbon (2 g/0,5 g).

La crème obtenue a les mêmes propriétés que celle de l'exemple 7.

EXEMPLE 11 : COMPOSITION DEODORANTE

On prépare une composition comme dans l'exemple 7, sauf que l'on utilise un lipide non-ionique de for-

mule :

$$C_{16}H_{33}-[OCH_2-CHOH-CH_2]_2-OH$$

La crème obtenue a les mêmes propriétés que celle de l'exemple 7.

EXEMPLE 12 : COMPOSITION DEODORANTE

On prépare une composition comme dans l'exemple 1, sauf que l'on utilise, comme lipide amphiphile, un lipide non-ionique de formule :

$$C_{15}H_{31}-CO-[OCH_2-CHOH-CH_2]_2-OH$$

La crème obtenue a les mêmes propriétés que celle de l'exemple 7.

EXEMPLE 13 : COMPOSITION DEODORANTE

Dans une première étape, on fond, en agitant doucement à une température de 80 - 90°C, un mélange de 3 g de lipide non-ionique de formule :

$$C_{12}H_{25}-[OC_2H_3(R_4)]-O-[C_3H_5-(OH)-O]_{\bar{n}}-H$$

formule dans laquelle :

- $C_3H_5(OH)$ O- est constitué par un mélange des structures :

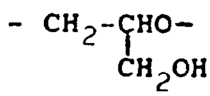

et

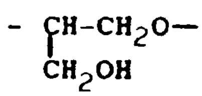

- O-$C_2H_5(R_4)$- est constitué par un mélange des structures :

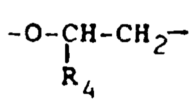

et

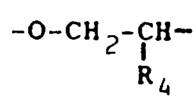

- $\bar{n} = 6$
- $R_4$ est un mélange de radicaux $C_{14}H_{29}$ et $C_{16}H_{33}$, avec 0,8 g de cholestérol. On obtient une dispersion de vésicules de lipides dans une phase aqueuse.

Dans une seconde étape, on additionne, à la phase aqueuse, goutte à goutte, 2 g d'huile essentielle de litsea cubeba en agitant à l'agitateur "ULTRA TURRAX"® pendant 5 minutes. Puis on ajoute un gel aqueux bien homogène constitué de 0,15 g de "CARBOPOL 940"® vendu par la société "GOODRICH", dissous dans

3 g d'eau renfermant des conservateurs et ajusté à pH 6 avec de la triéthanolamine et on complète à 100 g avec de l'eau.

La crème obtenue a les mêmes propriétés que celle de l'exemple 7.

EXEMPLE 14 : COMPOSITION DEODORANTE

Dans une première étape, on fond, en agitant doucement à une température de 80 - 90°C, un mélange de 3,6 g du lipide non-ionique défini et utilisé à l'exemple 7 avec 3,6 g de cholestérol et 0,4 g de dicétylphosphate.

On introduit dans le mélange fondu 15,2 g d'eau portée à 90°C contenant un conservateur et l'on mélange pendant environ 6 à 8 minutes au moyen d'un agitateur "ULTRA TURRAX"®. A la phase ainsi obtenue, on ajoute 22,8 g d'eau à température ambiante, puis on homogénéise le mélange à l'agitateur "ULTRA TURRAX"® pendant encore environ 6 à 8 minutes avant de laisser revenir le mélange à la température ambiante. On obtient une dispersion de vésicules de lipides dans une phase aqueuse.

Dans une seconde étape, on additionne, à la phase aqueuse, goutte à goutte, 5 g d'huile essentielle de melaleuca alternifolia en agitant à l'agitateur "ULTRA TURRAX"® pendant 5 minutes. Puis on ajoute un gel aqueux bien homogène constitué de 0,3 g de "CARBOPOL 940"® vendu par la société "GOODRICH", dissous dans 6 g d'eau renfermant des conservateurs et ajusté à pH 6 avec de la triéthanolamine et on complète à 100 g avec de l'eau.

La crème obtenue a les mêmes propriétés que celle de l'exemple 7.

EXEMPLE 15 : COMPOSITION INSECTIFUGE

Dans une première étape, on fond en agitant doucement à une température de 85°C, 1,6 g de phytostérol polyoxyéthyléné à 5 moles d'oxyde d'éthylène vendu par la Société HENKEL sous la dénomination "GENEROL 122 E 5"®. Puis au mélange fondu, on additionne 2,4 g de lécithine hydrogénée à 30-35 % de phosphatidylcholine hydrogénée vendue par la Société NIKKO sous la dénomination "LECINOL S 10"® et ceci, jusqu'à homogénéisation parfaite (5 minutes).

On introduit dans le mélange fondu 12,3 g d'eau portée à 80°C contenant un conservateur et l'on mélange pendant environ 5 minutes, au moyen d'un agitateur "ULTRA TURRAX"® puis on laisse gonfler le mélange pendant une heure. A la phase ainsi obtenue, on ajoute 18,4 g d'eau à 20°C ; on agite le mélange quelques minutes au moyen d'un agitateur "ULTRA TURRAX"®. On obtient une dispersion de vésicules dans une phase aqueuse.

Dans une seconde étape, on additionne goutte à goutte 6 g d'huile essentielle de Laurier noble et 6 g d'huile essentielle de Lavandin en agitant à l'agitateur "ULTRA TURRAX"®. Puis, on ajoute un gel aqueux bien homogène constitué de 0,3 g d'agent épaississant vendu sous la dénomination commerciale "CARBOPOL 940"® par la Société GOODRICH dissous dans 6 g d'eau renfermant des conservateurs et ajusté à pH 6,5 avec 2-amino 2 méthyl 1-propanol et on complète à 100 g avec de l'eau.

On obtient une crème ayant les mêmes propriétés que celle de l'exemple 1.

EXEMPLE 16 : COMPOSITION INSECTIFUGE

Dans une première étape, on fond en agitant doucement à une température de 60°C, 6 g de lécithine de soja à 75 % de phosphatidylcholine vendue par la société SEPPIC sous la dénomination commerciale "LIPOID S 75"®.

On introduit dans le mélange fondu 19,2 g d'eau portée à 80°C contenant un conservateur et l'on mélange pendant environ 5 minutes, au moyen d'un agitateur "ULTRA TURRAX"®; on laisse gonfler le mélange pendant une heure. A la phase ainsi obtenue, on ajoute 28,8 g d'eau à 20°C ; on agite le mélange quelques minutes au moyen d'un agitateur "ULTRA TURRAX"®; on complète par 13,9 g d'eau à 20°C sous agitation avec ce même agitateur. On obtient des vésicules dispersées dans une phase aqueuse.

Dans une seconde étape, on additionne goutte à goutte 4 g d'huile essentielle d'eucalyptus et 4 g d'huile essentielle de pin en agitant au moyen de l'agitateur "ULTRA TURRAX"®. Puis on ajoute un gel aqueux bien homogène constitué de 0,2 g d'agent épaississant vendu sous la dénomination commerciale "CARBOPOL 940"® par la Société GOODRICH dissous dans 4 g d'eau renfermant des conservateurs et ajusté à pH 6,5 avec 2-amino 2-méthyl 1-propanol ; on ajoute ensuite 0,1 g de 2,6 bis (1,1-diméthyl éthyl) -4-méthyl phénol (antioxydant) et on complète à 100 g avec de l'eau.

On obtient une crème ayant les mêmes propriétés que celle de l'exemple 1.

EXEMPLE 17 : COMPOSITION DEODORANTE

Dans une première égape, on fond en agitant doucement à une température de 85°C, 0,8 g de phytostérol polyoxyéthyléné à 5 moles d'oxyde d'éthylène vendu par la Société HENKEL sous la dénomination "GENEROL 122 E 5"®. Puis au mélange fondu, on additionne 1,2 g de lécithine hydrogénée à 30-35 % de phosphatidylcholine hydrogénée vendue par a société NIKKO sous la dénomination "LECINOL S 10"® et ceci, jusqu'à homogénéisation parfaite (5 minutes).

On introduit dans le mélange fondu 6,13 g d'eau portée à 80°C contenant un conservateur et l'on mélange pendant environ 5 minutes, au moyen d'un agitateur "ULTRA TURRAX"® puis on laisse gonfler le mélange pendant une heure. A la phase ainsi obtenue, on ajoute 9,2 g d'eau à 20°C ; on agite le mélange quelques minutes au moyen d'un agitateur "ULTRA TURRAX"® ; on obtient une dispersion de vésicules dans une phase aqueuse.

Dans une seconde étape, on additionne goutte à goutte 1 g d'huile essentielle de Santal et 1 g d'huile essentielle de Coriandre en agitant à l'agitateur "ULTRA TURRAX"®. Puis, on ajoute un gel aqueux bien homogène constitué de 0,15 g d'agent épaississant vendu sous la dénomination commerciale "CARBOPOL 934"® par la Société GOODRICH dissous dans 3 g d'eau renfermant des conservateurs et ajusté à pH 6 avec 2-amino 2-méthyl 1-propanol et on complète à 100 g avec de l'eau.

On obtient une crème ayant les mêmes propriétés que celle de l'exemple 7.

EXEMPLE 18 : COMPOSITION DEODORANTE

Dans une première étape, on fond en agitant doucement à une température de 60°C, 1,5 g de lécithine de soja à 75 % de phosphatidylcholine vendue par la Société SEPPIC sous la dénomination commerciale "LIPOID S 75".

On introduit dans le mélange fondu 4,8 g d'eau portée à 80°C contenant un conservateur et l'on mélange pendant environ 5 minutes, au moyen d'un agitateur "ULTRA TURRAX"® ; on laisse gonfler le mélange pendant une heure. A la phase ainsi obtenue, on ajoute 7,2 g d'eau à 20°C ; on agite le mélange quelques minutes au moyen d'un agitateur "ULTRA TURRAX"® ; on complète par 3,5 g d'eau à 20°C sous agitation avec ce même agitateur. On obtient des vésicules dispersées dans une phase aqueuse.

Dans une seconde étape, on additionne goutte à goutte 1,5 g d'huile essentielle de Sarriette et 1,5 g d'huile essentielle de Vétyver d'Haïti en agitant au moyen de l'agitateur "ULTRA TURRAX"®. Puis on ajoute un gel aqueux bien homogène constitué de 0,2 g d'agent épaississant vendu sous la dénomination commerciale "CARBOPOL 940"® par la Société GOODRICH dissous dans 4 g d'eau renfermant des conservateurs et ajusté à pH 6 avec 2-amino 2-méthyl 1-propanol ; on complète à 100 g avec de l'eau.

On obtient une crème ayant les memes propriétés que celle de l'exemple 7.

EXEMPLE 19 : ESSAIS COMPARATIFS

On a comparé l'action sur différentes bactéries et moisissures d'une composition C préparée comme dans l'exemple 7, contenant 0,95% du même lipide non-ionique qu'à l'exemple 7, 0,95% de cholestérol, 0,1% de dicétylphosphate, et 1 % en poids d'huile essentielle de litsea cubeba, avec l'action d'une solution éthanolique D contenant 2 % en poids de la même huile essentielle.

Dans ces essais, on a mesuré par la méthode des cupules, sur des cultures microbiennes, le diamètre des zones d'inhibition, puis on a calculé les résultats après déduction de l'action propre du support. On peut voir sur le tableau I ci-après que, dans de nombreux cas, l'activité de la composition C est voisine de celle de la solution alcoolique D. Dans les compositions selon l'invention, l'activité propre à l'huile essentielle est, par conséquent, exaltée, ce qui permet d'obtenir, en l'absence d'éthanol, des compositions ayant des propriétés antibactériennes et fongicide meilleures que les solutions alcooliques classiques correspondantes avec une quantité moindre d'huile essentielle.

TABLEAU I

| Germes | DIAMETRE DES ZONES D'INHIBITION EN MM | | | | RESULTATS APRES DEDUCTION DE L'EFFET SUPPORT | |
|---|---|---|---|---|---|---|
| | Ethanol | Solution D | Composition C | Vésicules seules | Support vésicules | Support alcoolique |
| Bactéries | | | | | | |
| Gram + | | | | | | |
| Staphylococcus aureus | 11,5 | 21,0 | 15,0 | 0,0 | 15,0 | 9,5 |
| Staphylococcus epidermidis | 11,5 | 15,0 | 15,0 | 0,0 | 15,0 | 3,5 |
| Sarcina lutea | 10,0 | 14,0 | 12,0 | 0,0 | 12,0 | 4,0 |
| Bacillus subtilis | 11,5 | 18,8 | 17,0 | 0,0 | 17,0 | 7,3 |
| Micrococcus luteus | 9,5 | 13,0 | 10,5 | 0,0 | 10,5 | 3,5 |
| Streptococcus faecalis | 9,5 | 13,5 | 11,5 | 0,0 | 11,5 | 4,0 |
| Moisissures | | | | | | |
| Mucor mucedo | 12,5 | 25,0 | 27,0 | 11,0 | 16,0 | 12,5 |
| Rhyzopus nigricans | 14,5 | 35 | 30,0 | 0,0 | 30,0 | 20,5 |
| Penicillium notatum | 11,0 | 28,0 | 18,0 | 0,0 | 18,0 | 17,0 |

EXEMPLE 20 : ESSAIS COMPARATIFS

On a préparé différentes compositions insectifuges vésiculées à partir du lipide amphiphile non ionique de l'exemple 6 et selon le procédé décrit dans cet exemple, ainsi qu'une composition insectifuge ne contenant que l'huile essentielle en solution alcoolique.

| Compositions | A | B | C | D | E | F |
|---|---|---|---|---|---|---|
| Lipide nonionique de l'exemple 6 | 0,9 | 7,2 | 14,4 | 3,6 | 6 | |
| "Acylglutamate HS11"® défini à l'exemple 2 | 0,1 | 0,8 | 1,6 | 0,4 | | |
| Cholestérol | | | | | 1,6 | |
| Huile essentielle de citronnelle | 10 | 10 | 3,5 | 3,5 | 10 | 12,5 |
| "Carbopol 934"® défini à l'exemple 1 | 0,3 | 0,3 | 0,3 | 0,3 | 0,3 | |
| 2-amino 2-méthyl 1-propanol (qs pH) | 6,5 | 6,5 | 6,5 | 6,5 | 6,5 | |
| Conservateurs | qs | qs | qs | qs | qs | |
| Eau qsp | 100 | 100 | 100 | 100 | 100 | |
| Ethanol absolu qsp | | | | | | 100 |

1 - Justification de la borne inférieure du rapport (lipides totaux (L)/huile essentielle (H)).

On étudie la vitesse d'évaporation à la balance à extrait sec des compositions A et B.

On introduit 2 g de composition à étudier sur un support inerte à une température de 55°C. On pèse alors jusqu'à obtention d'un poids constant. Le temps écoulé correspond donc au temps nécessaire pour obtenir un extrait sec. On répète l'opération 5 fois pour chaque composition.

On obtient les résultats suivants :

. Composition A : extrait sec obtenu en un temps moyen de 21 minutes.

. Composition B : extrait sec obtenu en un temps moyen de 32 minutes.

On en conclut qu'avec la composition B (L/H=0,8) l'évaporation est plus lente qu'avec la composition A (L/H=0,1). On note une différence de 50 % au niveau de la vitesse d'évaporation. Ce test a été réalisé à une température de 55°C pour accélérer l'évaporation ; à une température égale à celle de la surface de la peau, la différence serait encore plus marquée.

L'effet insectifuge des compositions selon l'invention (L/H>0,2) est donc plus durable que lorsque le rapport L/H est inférieur à 0,2.

2 - Justification de la borne supérieure du rapport L/H.

On a comparé les propriétés cosmétiques des deux compositions suivantes :

. Composition C : L/H = 4,75

. Composition D : L/H = 1,14

Le test a été réalisé en apparié sur 5 modèles M1 à M5. Les compositions C et D sont appliquées sur la face interne des avant-bras.

Lors de l'application, différents critères sont notés par le testeur :

. glissant

. gras

. collant

. frein (difficulté d'étalement)

. pénétration.

Une fois la pénétration des compositions effectuée, les critères suivants sont appréciés :

. douceur de la peau

. gras

. collant

Le testeur porte enfin une appréciation globale notée de 0 à 5 et appelée dans le tableau "préférence".

Chaque critère est noté de 0 à 5 : note 0=mauvais,note 5=bon.

Les résultats obtenus sont présentés dans le tableau II.

On en conclut que la composition D (L/H<4) est nettement préférée à la composition C (L/H>4) qui est grasse et collante. Les compositions selon l'invention doivent donc avoir un rapport L/H< 4 pour présenter de bonnes propriétés cosmétiques.

3 - Comparaison de la forme vésiculée par rapport à la forme alcoolique.

Un test d'irritation a été effectué avec les deux compositions suivantes :

. Composition E : identique à celle de l'exemple 6 (7,6 % de lipides)

. Composition F : huile essentielle en solution éthanolique.

L'étude a été réalisée sur deux modèles. On applique sur la face interne des avant-bras 50µl de composition E ou F, sous patch test occlusif pendant 24 heures. Les quantités d'huiles essentielles contenues dans 50µl de composition E ou F sont égales à 100 g/l. La densité de la composition E est de 1 ; celle de la composition F est de 0,8.

On constate que la forme vésiculée (composition E) provoque une très faible irritation par rapport à la forme alcoolique (composition F), qui entraîne une forte irritation. La composition E selon l'invention est donc mieux tolérée.

TABLEAU II

| | Composit.C | Ecart type | Composit.D | Ecart type | Composition C M1 | M2 | M3 | M4 | M5 | Composition D M1 | M2 | M3 | M4 | M5 |
|---|---|---|---|---|---|---|---|---|---|---|---|---|---|---|
| Glissant | 4,00 | 0,35 | 4,20 | 0,27 | 4 | 3,5 | 4,5 | 4 | 4 | 4,5 | 4 | 4 | 4,5 | 4 |
| Gras | 2,80 | 0,76 | 3,30 | 0,57 | 2 | 3,5 | 3 | 3,5 | 2 | 3,5 | 3 | 3,5 | 4 | 2,5 |
| Collant | 1,70 | 0,57 | 2,50 | 0,61 | 1,5 | 2,5 | 2 | 1,5 | 1 | 2,5 | 3,5 | 2,5 | 2 | 2 |
| Frein | 2,60 | 0,42 | 2,10 | 1,08 | 2,5 | 3 | 3 | 2,5 | 2 | 1,5 | 3,5 | 3 | 1,5 | 1 |
| Pénétration | 3,00 | 0,35 | 3,50 | 0,50 | 3,5 | 3 | 3 | 3 | 2,5 | 3 | 4 | 4 | 3,5 | 3 |
| Douceur | 2,30 | 0,84 | 3,50 | 0,50 | 3 | 2,5 | 3 | 2 | 1 | 4 | 3,5 | 4 | 3 | 3 |
| Gras | 3,90 | 0,42 | 4,30 | 0,27 | 4 | 3,5 | 3,5 | 4,5 | 4 | 4,5 | 4,5 | 4,5 | 4 | 4 |
| Collant | 2,60 | 0,65 | 4,00 | 0,35 | 2 | 3,5 | 3 | 2,5 | 2 | 4 | 4,5 | 4 | 3,5 | 4 |
| Préférence | 2,40 | 0,89 | 3,70 | 0,45 | 2 | 3 | 3 | 3 | 1 | 3,5 | 4 | 4 | 4 | 3 |

## Revendications

1. Composition cosmétique pour application topique contenant des huiles essentielles ayant une activité antibactérienne, antifongique ou insectifuge, caractérisée par le fait qu'elle contient, dans une phase aqueuse continue de dispersion :
   a) des vésicules préparées à partir d'une phase lipidique comprenant au moins un lipide amphiphile ionique ou non-ionique éventuellement associée à au moins un additif stabilisant, et
   b) au moins une huile naturelle ou synthétique sous forme de gouttelettes dispersées, l'huile essentielle étant présente à raison de 0,1 à 16 % en poids par rapport au poids total de la composition, et le rapport pondéral de la phase lipidique à l'huile essentielle étant supérieur à 0,2 et inférieur ou égal à 4.

2. Composition selon la revendication 1, caractérisée par le fait que les vésicules ont une dimension moyenne comprise entre 10 et 1000 nm.

3. Composition selon les revendications 1 ou 2, caractérisée par le fait que les gouttelettes d'huile(s) essentielle(s) ont une dimension moyenne comprise entre 100 et 10000 nm.

4. Composition selon l'une des revendications 1 à 3, caractérisée par le fait que le lipide amphiphile est un lipide non-ionique constitué par un dérivé de polyglycérol, linéaire ou ramifié, de formule (I) :

$$RO-\left[C_3H_5(OH)O\right]_{\bar{n}}-H \qquad (I)$$

formule dans laquelle :
- $C_3H_5(OH)O$- représente les structures suivantes prises en mélange ou séparément :

  - $CH_2$-CHOH-$CH_2$O-

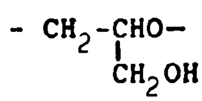

et

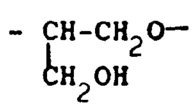

- $\bar{n}$ est une valeur moyenne comprise entre 2 et 6, et
- R est :
  a) soit une chaîne aliphatique $R_1$ ou un reste $R_2CO$, $R_1$ étant un radical aliphatique, linéaire ou ramifié en $C_{12}$-$C_{18}$ et $R_2$ un radical aliphatique, linéaire ou ramifié, en $C_{11}$-$C_{17}$ ;
  b) soit

$$R_3\left[O-C_2H_3(R_4)\right]-$$

où -O-$C_2H_3(R_4)$- représente les structures suivantes prises en mélange ou séparément :

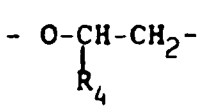

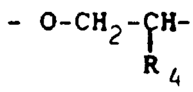

- $R_3$ étant un radical $R_1$ ou $R_2CO$,
- $R_4$ étant un radical $R_1$, et
- $R_1$ et $R_2$ ayant les significations données ci-dessus

**5.** Composition selon l'une des revendications 1 à 4, caractérisée par le fait que l'additif stabilisant est choisi dans le groupe formé par les stérols et les stabilisants anioniques.

**6.** Composition selon la revendication 5, caractérisée par le fait que le stérol est le cholestérol.

**7.** Composition selon la revendication 5, caractérisée par le fait que le stabilisant anionique est choisi parmi les sels monosodiques ou disodiques des acyl-glutamates, où le radical acyle est en $C_{14}$-$C_{22}$ et les esters phosphoriques d'alcools gras en $C_{12}$-$C_{22}$.

**8.** Composition selon l'une des revendications 1 à 7, caractérisée par le fait que la phase lipidique représente de 0,5 à 16 % en poids par rapport au poids total de la composition.

**9.** Composition selon l'une des revendications 1 à 8, caractérisée par le fait que le(s) lipide(s) amphiphile(s) ionique(s) ou non-ionique(s) de la phase lipidique représente(nt), de préférence, de 0,2 à 16 % en poids par rapport au poids total de la composition.

**10.** Composition selon l'une des revendications 1 à 9, caractérisée par le fait que l'additif stabilisant représente moins de 10% en poids du poids total de la composition.

**11.** Composition selon l'une des revendications 1 à 10, caractérisée par le fait que la phase aqueuse de dispersion contient au moins un additif choisi dans le groupe formé par les conservateurs, les parfums, les agents acidifiants, les agents alcalinisants, les stabilisants, les colorants et les agents épaississants.

**12.** Composition selon l'une des revendications 1 à 11, destinée à constituer un déodorant corporel, caractérisée par le fait que l'huile essentielle est choisie parmi celles ayant une activité antibactérienne et/ou antifongique.

**13.** Composition selon la revendication 12, caractérisée par le fait que l'huile essentielle est choisie parmi celles ayant une activité contre les bactéries Gram-positif.

**14.** Composition selon la revendication 12, caractérisée par le fait que l'huile essentielle est choisie dans le groupe formé par les huiles de litsea cubeba, de sarriette, de vétivers (Bourbon, Java, Palmarosa, et Haïti), de santal, de melaleuca, de camomille, de bois de rose du Brésil, de cannelle (Ceylan et Chine), de zeste de citron, de lavandin, de menthe poivrée, de thym, de patchouli, de linalol et de coriandre.

**15.** Composition selon l'une des revendications 12 à 14, caractérisée par le fait que 1'(ou les) huile(s) essentielle(s) est (ou sont) présente(s) à raison de 0,2 à 10 % en poids par rapport au poids total de la composition.

**16.** Composition selon l'une des revendications 1 à 11, destinée à constituer un insectifuge corporel, caractérisée par le fait que l'huile essentielle est choisie parmi celles ayant une activité insectifuge.

**17.** Composition selon la revendication 16, caractérisée par le fait que l'huile essentielle est choisie dans le groupe formé par les huiles de citronnelle, de géranium, de lavande, de lavandin, de pins, d'eucalyptus et de laurier noble.

**18.** Composition selon l'une des revendications 16 ou 17, caractérisée par le fait que l' (ou les) huile(s) essentielle(s) est (ou sont) présente(s) à raison de 1 à 12 % en poids par rapport au poids total de la composition.

## Claims

1. Cosmetic composition for topical application containing essential oils having antibacterial, antifungal or insect repellent activity, characterized by the fact that it contains, in a continuous aqueous dispersion phase:

   a) vesicles prepared from a lipid phase comprising at least one ionic or nonionic amphiphilic lipid optionally combined with at least one stabilising additive, and

   b) at least one natural or synthetic oil in the form of dispersed droplets, the essential oil being present in an amount of 0.1 to 16 % by weight relative to the total weight of the composition, and the weight ratio of the lipid phase to the essential oil being greater than 0.2 and less than or equal to 4.

2. Composition according to Claim 1, characterized by the fact that the vesicles have a mean size of between 10 and 1,000 nm.

3. Composition according to Claims 1 or 2, characterized by the fact that the droplets of essential oil(s) have a mean size of between 100 and 10,000 nm.

4. Composition according to one of Claims 1 to 3, characterized by the fact that the amphiphilic lipid is a nonionic lipid consisting of a linear or branched polyglycerol derivative of formula (I):

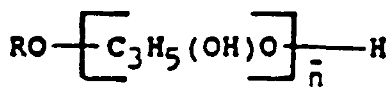

in which formula:

- $-C_3H_5(OH)O-$ represents the following structures taken together or separately:

  $- CH_2-CHOH-CH_2O-$

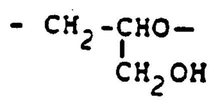

and

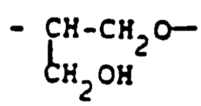

- $\bar{n}$ is a mean value between 2 and 6, and
- R is :

  a) either an aliphatic chain $R_1$ or a residue $R_2CO$, $R_1$ being a linear or branched $C_{12}$-$C_{18}$ aliphatic radical and $R_2$ a linear or branched $C_{11}$-$C_{17}$ aliphatic radical;

  b) or

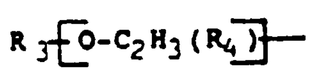

where $-O-C_2H_3(R_4)-$ represents the following structures taken together or separately:

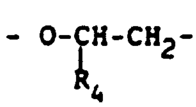

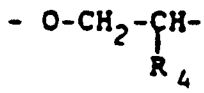

- $R_3$ being a radical $R_1$ or $R_2CO$,
- $R_4$ being a radical $R_1$, and
- $R_1$ and $R_2$ having the meanings given above.

5. Composition according to one of Claims 1 to 4, characterized by the fact that the stabilizing additive is chosen from the group consisting of sterols and anionic stabilizers.

6. Composition according to Claim 5, characterized by the fact that the sterol is cholesterol.

7. Composition according to Claim 5, characterized by the fact that the anionic stabilizer is chosen from the monosodium or disodium salts of acyl glutamates, where the acyl radical is a $C_{14}$-$C_{22}$ radical and the phosphoric esters of fatty alcohols a $C_{12}$-$C_{22}$.

8. Composition according to one of Claims 1 to 7, characterized by the fact that the lipid phase represents 0.5 to 16 % by weight relative to the total weight of the composition.

9. Composition according to one of Claims 1 to 8, characterized by the fact that the ionic or nonionic amphiphilic lipid(s) of the lipid phase preferably represent(s) 0.2 to 16 % by weight relative to the total weight of the composition.

10. Composition according to one of Claims 1 to 9, characterized by the fact that the stabilizing additive represents less than 10 % by weight of the total weight of the composition.

11. Composition according to one of Claims 1 to 10, characterized by the fact that the aqueous dispersion phase contains at least one additive chosen from the group consisting of preservatives, perfumes, acidifying agents, alkalinizing agents, stabilizers, colorants and thickening agents.

12. Composition according to one of Claims 1 to 11, intended to constitute a body deodorant, characterized by the fact that the essential oil is chosen from those having an antibacterial and/or antifungal activity.

13. Composition according to Claim 12, characterized by the fact that the essential oil is chosen from those having an activity against Gram-positive bacteria.

14. Composition according to Claim 12, characterized by the fact that the essential oil is chosen from the group consisting of litsea, cubeb, savory, vetiver (Bourbon, Java, Palmarosa and Haïti), sandalwood, melaleuca, chamomile, Brazil rosewood, cinnamon (Ceylon and China), lemon zest, lavendin, peppermint, thyme, patchouli, linalol and coriander oils.

15. Composition according to one of Claims 12 to 14, characterized by the fact that the essential oil(s) is (or are) present in an amount of 0.2 to 10 % by weight relative to the total weight of the composition.

16. Composition according to one of Claims 1 to 11, intended to constitute a body insect repellent, characterized by the fact that the essential oil is chosen from those having an insect repellent activity.

17. Composition according to Claim 16, characterized by the fact that the essential oil is chosen from the group consisting of citronella, geranium, lavender, lavandin, pine, eucalyptus and noble laurel oils.

18. Composition according to either of Claims 16 and 17, characterized by the fact that the essential oil(s) is (or are) present in an amount of 1 to 12 % by weight relative to the total weight of the composition.

**Patentansprüche**

1. Kosmetisches Mittel zur topischen Anwendung, enthaltend ätherische Öle mit antibakterieller, antifungischer oder insektifuger Aktivität, dadurch gekennzeichnet, daß es in einer wäßrigen, kontinuierlichen Dispersionsphase:

a) Versikel, hergestellt aus einer Lipidphase, umfassend wenigstens ein amphiphiles ionisches oder nicht-ionisches Lipid, gegebenenfalls in Kombination mit wenigstens einem stabilisierenden Zusatz, und

b) wenigstens ein natürliches oder synthetisches Öl in Form von dispergierten Tröpfchen enthält, wobei das ätherische Öl in einem Anteil von 0,1 bis 16 Gew.-%, bezogen auf das Gesamtgewicht des Mittels, enthalten ist, und das Gewichtsverhältnis von Lipidphase zum ätherischen Öl größer als 0,2 und kleiner oder gleich 4 ist.

**2.** Mittel nach Anspruch 1, dadurch gekennzeichnet, daß die Vesikel eine mittlere Größe im Bereich von 10 bis 1000 nm besitzen.

**3.** Mittel nach Anspruch 1 oder 2, dadurch gekennzeichnet, daß die Tröpfchen des (der) ätherischen Öls (Öle) eine mittlere Größe im Bereich von 100 bis 10000 nm besitzen.

**4.** Mittel nach einem der Ansprüche 1 bis 3, dadurch gekennzeichnet, daß das amphiphile Lipid ein nicht-ionisches Lipid ist, bestehend aus einem geradkettigen oder verzweigten Polyglycerinderivat der Formel (I) :

$$RO-[C_3H_5(OH)O]_{\bar{n}}-H \qquad (I)$$

worin:
$-C_3H_5(OH)O-$ für eine der folgenden Strukturen oder eine Mischung davon steht:

$- CH_2-CHOH-CH_2O-$

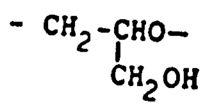

und

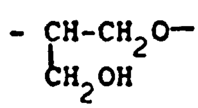

worin $\bar{n}$ für einen Mittelwert von 2 bis 6 steht, und
R für

a) eine aliphatische Kette $R_1$ oder für einen Rest $R_2CO$ steht, wobei $R_1$ für einen geradkettigen oder verzweigten, aliphatischen $C_{12}$ - $C_{18}$-Rest und $R_2$ für einen geradkettigen oder verzweigten, $C_{11}$ - $C_{17}$-Rest steht; oder

b) für

$$R_3-[O-C_2H_3(R_4)]-$$

steht,
worin $-O-C_2H_3(R_4)-$ für eine der folgenden Strukturen oder eine Mischung davon steht:

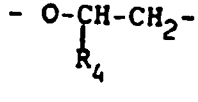

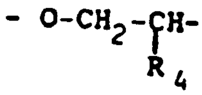

$R_3$ für einen Rest $R_1$ oder $R_2CO$ steht;
$R_4$ für einen Rest $R_1$ steht und
$R_1$ und $R_2$ die oben angegebenen Bedeutungen besitzen.

**5.** Mittel nach einem der Ansprüche 1 bis 4, dadurch gekennzeichnet, daß der stabilisierende Zusatz ausgewählt ist unter Sterolen und anionischen Stabilisatoren.

**6.** Mittel nach Anspruch 5, dadurch gekennzeichnet, daß das Sterol Cholesterol ist.

**7.** Mittel nach Anspruch 5, dadurch gekennzeichnet, daß der anionische Stabilisator ausgewählt ist unter Mononatrium- oder Dinatriumsalzen von Acylglutamaten, wobei der Acylrest ein $C_{14}$-$C_{22}$-Rest ist, und Phosphorsäureestern von $C_{12}$-$C_{22}$-Fettalkoholen.

**8.** Mittel nach einem der Ansprüche 1 bis 7, dadurch gekennzeichnet, daß die Lipidphase in einem Anteil von 0,5 bis 16 Gew.-%, bezogen auf das Gesamtgewicht des Mittels, enthalten ist.

**9.** Mittel nach einem der Ansprüche 1 bis 8, dadurch gekennzeichnet, daß das (die) amphiphile(n) ionische(n) oder nicht-ionische(n) Lipid(e) der Lipidphase vorzugsweise in einem Anteil von 0,2 bis 16 Gew.-%, bezogen auf das Gesamtgewicht des Mittels, enthalten ist (sind).

**10.** Mittel nach einem der Ansprüche 1 bis 9, dadurch gekennzeichnet, daß der stabilisierende Zusatz in einem Anteil von weniger als 10 Gew.-%, bezogen auf das Gesamtgewicht des Mittels, enthalten ist.

**11.** Mittel nach einem der Ansprüche 1 bis 10, dadurch gekennzeichnet, daß die wäßrige Dispersionsphase wenigstens einen Zusatz enthält, der ausgewählt ist unter Konservierungsmitteln, Parfums, acidifizierenden Mitteln, alkalisierenden Mitteln, Stabilisatoren, Farbstoffen und Verdickungsmitteln.

**12.** Mittel nach einem der Ansprüche 1 bis 11, bestimmt zur Herstellung eines Körperdeodorants, dadurch gekennzeichnet, daß das ätherische Öl ausgewählt ist unter Ölen mit antibakterieller und/oder fungizider Aktivität.

**13.** Mittel nach Anspruch 12, dadurch gekennzeichnet, daß das ätherische Öl ausgewählt ist unter Ölen mit Aktivität gegen Gram-positive Bakterien.

**14.** Mittel nach Anspruch 12, dadurch gekennzeichnet, daß das ätherische Öl ausgewählt ist unter Ölen aus Litsea Cubeba, Pfefferkraut, Vétiver (Bourbon, Java, Palmarosa und Haiti), Sandelholz, Melaleuca, Kamille, Holz der brasilianischen Rose, Zimt (Ceylon und China), Zitronenschale (zeste de citron), Lavandin, Pfefferminze, Thymian, Patchouli, Linalol und Koriander.

**15.** Mittel nach einem der Ansprüche 12 bis 14, dadurch gekennzeichnet, daß das (die) ätherische(n) Öl(e) in einem Anteil von 0,2 bis 10 Gew.-%, bezogen auf das Gesamtgewicht des Mittels, enthalten ist (sind).

**16.** Mittel nach einem der Ansprüche 1 bis 11, bestimmt zur Herstellung eines insektifugen Mittels für den Körper, dadurch gekennzeichnet, daß das ätherische Öl ausgewählt ist unter Ölen mit insektifuger Aktivität.

**17.** Mittel nach Anspruch 16, dadurch gekennzeichnet, daß das ätherische Öl ausgewählt ist unter Citronella-, Geranium-, Lavendel-, Lavandin-, Kiefern-, Eukalyptus- und Lorbeer (laurier noble)-Öl.

**18.** Mittel nach einem der Ansprüche 16 oder 17, dadurch gekennzeichnet, daß das (die) ätherische(n) Öl(e) in einem Anteil von 1 bis 12 Gew.-%, bezogen auf das Gesamtgewicht des Mittels, enthalten ist (sind).